# EUROPEAN PATENT APPLICATION

(11) **EP 4 491 144 A1**
(43) Date of publication of application: **15.01.2025**
(21) Application number: 23306221.5
(22) Date of filing: 13.07.2023
(51) Int. Cl.: A61B 34/32

(54) **SURGICAL SYSTEM FOR OSTEOTOMY**

(71) Applicant: Ostesys, 38400 Saint-Martin-D'Hères (FR)
(72) Inventor: CHANRION, Marie-Anne, 38400 SAINT-MARTIN-D'HÈRES (FR); CHOPARD, Teddy, 38400 SAINT-MARTIN-D'HÈRES (FR); COSTA, Gilliane, 38400 SAINT-MARTIN-D'HÈRES (FR); ARFUSO, Maeva, 38400 SAINT-MARTIN-D'HÈRES (FR)
(74) Representative: Regimbeau

(57) **Abstract**

A surgical system (100) for osteotomy comprising:
a robotic arm (110);
a surgical tool (130);
a storage unit (140);
a tracking system (150); and
a control unit (120),
the control unit (120) being configured to:
compute a drilling axis of a screw hole, a depth of a screw hole and a cutting plane based on a pose of the implant (40) and a position of the plurality of screws (50) of a 3D bone model,
control the robotic arm (110) to:
align the surgical tool (130) with the cutting plane or the drilling axis based on the localization data provided by the tracking system (150); and
displace the surgical tool (130) along the predetermined drilling axis (20) until reaching the predetermined depth based on the localization data.

## Description

### TECHNICAL FIELD

The present subject matter relates generally to surgical systems, more particularly, surgical systems for osteotomies.

### BACKGROUND

In some osteotomies, a bone is cut and deformed in particular for correcting a limb misalignment by moving bone portions - obtained after cutting the bone - away or toward each other about a bony hinge to reach a deformed configuration. To maintain the bone in the deformed configuration, an implant may then be fixed to the bone portions with a plurality of screws in a plurality of respective screw holes previously drilled in the bone.

There exist several types of screws. For instance, screws may be mono-cortical or bi-cortical according to the number of threads engaged in the cortical bone. The biomechanically advantages of using bicortical screws encourages the surgeons to use more and more bicortical screws. To that end, some surgical systems may drill a screw hole through both sides of the bone to allow the surgeon to use a gauge to determine a length of the respective screw. The gauge is inserted into the screw hole and the surgeon slides a mobile part of the gauge until coming into contact with one side of the screw hole. The surgeon reads the gauge at this position to determine a length of the screw hole and then selects the length of the respective screw based on the previously determined length of the screw hole.

However, drilling through both sides of the bone to use such gauges may increase the risk of overdrilling, for example drilling soft tissues hidden behind the bone.

Accordingly, improving hole drilling in osteotomies would be useful.

### SUMMARY

According to a first aspect of the present disclosure, it is provided a surgical system for osteotomy of a first bone of a lower limb to correct a misalignment of the lower limb by moving a first bone portion of the first bone and a second bone portion of the first bone away or toward each other about a bony hinge to reach a targeted deformed configuration of the first bone achieving a corrected alignment of the lower limb, the first bone portion and the second bone portion being created after cutting the first bone the targeted deformed configuration of the first bone being maintained by an implant attached to the first bone portion and the second bone portion of the first bone with a plurality of screws in a plurality of respective screw holes, the lower limb comprising the first bone and a second bone articulated with the first bone, the surgical system comprising:
a storage unit;
a control unit configured to communicate with the storage unit;
a robotic arm, the control unit being configured to control the robotic arm;
a surgical tool configured to be attached to the robotic arm and to perform at least one of the following operations:
   drilling at least one screw hole in the first bone along a respective predetermined drilling axis and until a respective predetermined depth,
   performing a partial cut in the first bone along at least one predetermined cutting plane to create the first bone portion and the second bone portion,
the control unit being configured to compute the respective drilling axis, the respective predetermined depth and the predetermined cutting plane based on a planned position of the implant and a planned pose of the plurality of screws on a 3D model, the 3D model representing the first bone in the targeted deformed configuration,
the predetermined drilling axis, the predetermined depth and the predetermined cutting plane being stored in the storage unit,
a tracking system comprising, at least, a first tracker configured to be fixed to the robotic arm, a second tracker and a third tracker configured to be fixed respectively to the first bone and the second bone, the tracking system being configured to determine a relative pose of the robotic arm with respect to the first and the second bone, and to send corresponding localization data to the control unit,
the control unit being configured to control the robotic arm to:
   align the surgical tool with the predetermined cutting plane or the predetermined drilling axis based on the localization data provided by the tracking system; and
   displace the surgical tool along the predetermined drilling axis until reaching the predetermined depth based on the localization data.

By "pose", we mean both positions and orientations (i.e. six degrees of freedom in all) of an object, such as a tracker.

The 3D model may further comprise a bone volume and a bone surface of the first bone, the bone volume comprising a cortical bone and a cancellous bone of the first bone, the bone surface delimiting the bone volume, and the control unit may be further configured to compute the predetermined depth as a distance along the predetermined drilling axis from an entry point of the surgical tool on the bone surface to an end point located in the cortical bone opposite to the entry point with respect to the cancellous bone.

The storage unit may comprise a plurality of screw references, each screw reference defining a diameter and a length of a respective screw,
the control unit being further configured to select, for each screw hole, a screw reference whose diameter is less than or equal to a diameter of the screw hole and whose length corresponds to the respective predetermined depth of the screw hole.

The surgical system may further comprise a user interface coupled to the control unit,
the control unit being further configured to compute a representation of a relative pose of the surgical tool with respect to the predetermined cutting plane or with respect to the predetermined drilling axis, a representation of the predetermined drilling axis, a representation of the predetermined depth and/or a representation of the predetermined cutting plane on the model of the first bone,
the user interface being configured to display the representation.

The user interface may be further configured to display, for each screw hole, a representation of the selected screw reference.

The control unit may be further configured to allow an activation of the surgical tool only when the surgical tool is aligned with the predetermined cutting plane or the predetermined drilling axis.

The control unit may be further configured to control the robotic arm to displace and to align the surgical tool at a relative speed with respect to the predetermined cutting plane or the predetermined drilling axis between a predetermined minimum relative speed and a predetermined maximum relative speed.

The control unit may be further configured to control the robotic arm to displace and to align the surgical tool at a rotation speed between a predetermined minimum rotation speed and a predetermined maximum rotation speed.

The robotic arm may comprise at least six motorized joints, the control unit being further configured to control a motorized joint of the at least six motorized joints.

The surgical system may further comprise an input device configured to communicate with the control unit, the control unit being configured to receive an input from a user through the input device and to take into account said input to control the robotic arm and/or the surgical tool.

### BRIEF DESCRIPTION OF THE DRAWINGS

Further features, purposes and advantages will be apparent from the following description, which is purely illustrative and not limiting, and which should be read in conjunction with the drawings in which:
figure 1a to 1d are schematic views of a first bone and a second bone of a lower limb showing a partial cut for respectively a high-tibial osteotomy (figure 1a), a distal tibial osteotomy (figure 1b), a distal femoral osteotomy (figure 1c) and a proximal femoral osteotomy (figure 1d);
figures 2a and 2b illustrate different embodiments of a surgical system;
figures 3a and 3b illustrate a detailed embodiment of a robotic arm equipped with different surgical tools; and
figures 4a and 4b illustrate a fixation of an implant with respectively bi-cortical and mono-cortical screws.

### DETAILED DESCRIPTION

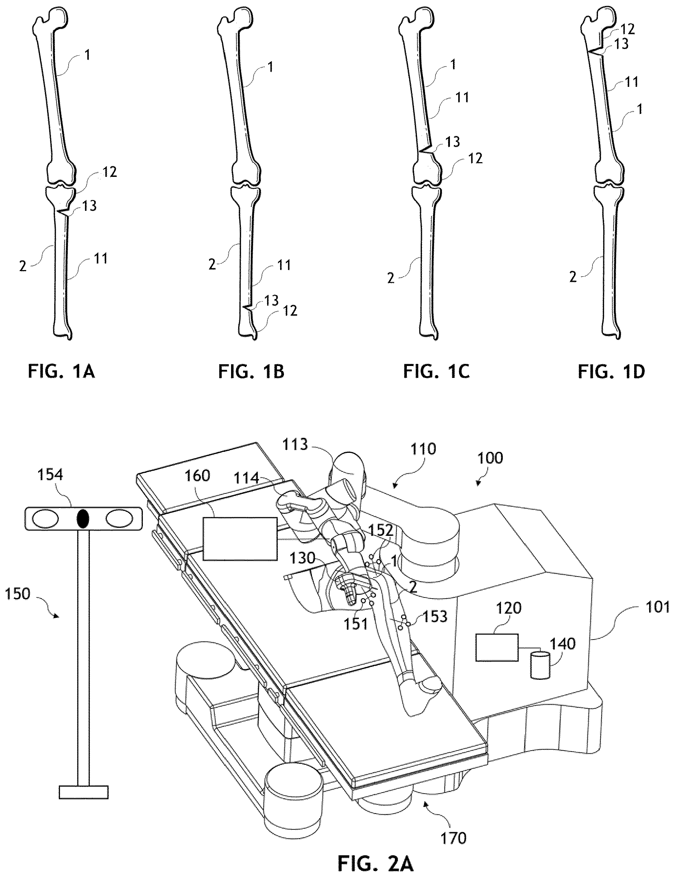

In reference to figures 1a to 1d, an osteotomy of a first 1, respectively second bone 2 of a lower limb is a surgical operation wherein the first 1, respectively second bone 2 is cut and reconfigured to correct a misalignment in the lower limb.

In the following description, it is assumed for the sake of conciseness that osteotomy is carried out on the first bone 1, but osteotomy may, in addition or alternatively, be carried out on the second bone 2.

In an open wedge osteotomy, a partial cut is made through the first bone 1 to obtain a first bone portion 11 and a second bone portion 12. The first and the second bone portions 11, 12 can then be moved away from each other about a bony hinge 13 to reach an opened targeted deformed configuration of the first bone 1 achieving a corrected alignment of the limb. In a closed wedge osteotomy, at least two partial cuts are performed in the first bone 1 to remove a wedge-shaped portion to obtain the first bone portion 11 and the second bone portion 12 that are moved toward each other about the bony hinge 13 to reach a closed targeted deformed configuration of the first bone 1 achieving a corrected alignment of the lower limb.

Depending on the correction to be achieved, the partial cut(s) may be performed in different locations of the limb. For example, the cut(s) may be performed in the tibia, adjacent to the knee, in case of a high-tibial osteotomy illustrated in figure 1a, or adjacent to the ankle, in case of a distal tibial osteotomy illustrated in figure 1b. The cut(s) may also be performed in the femur, adjacent to the knee, in case of a distal femoral osteotomy illustrated in figure 1c, or adjacent the hip, in case of a proximal femoral osteotomy illustrated in figure 1d. In some situations, cuts may be performed in both the tibia and the femur.

An implant is then fixed to the first bone 1 with a plurality of screws in a respective plurality of screw holes drilled in the first bone 1 to maintain the first bone 1 in the deformed configuration. The implant may be a locking implant or a non-locking implant. By "locking implant" we here mean that the implant comprises through holes comprising an inner thread configured to engage a respective outer thread of the screw, to hold the plurality of screws. By "non-locking implant" we here mean that the implant comprises through holes having a smooth inner surface, each screw passing through the implant without mechanical engagement.

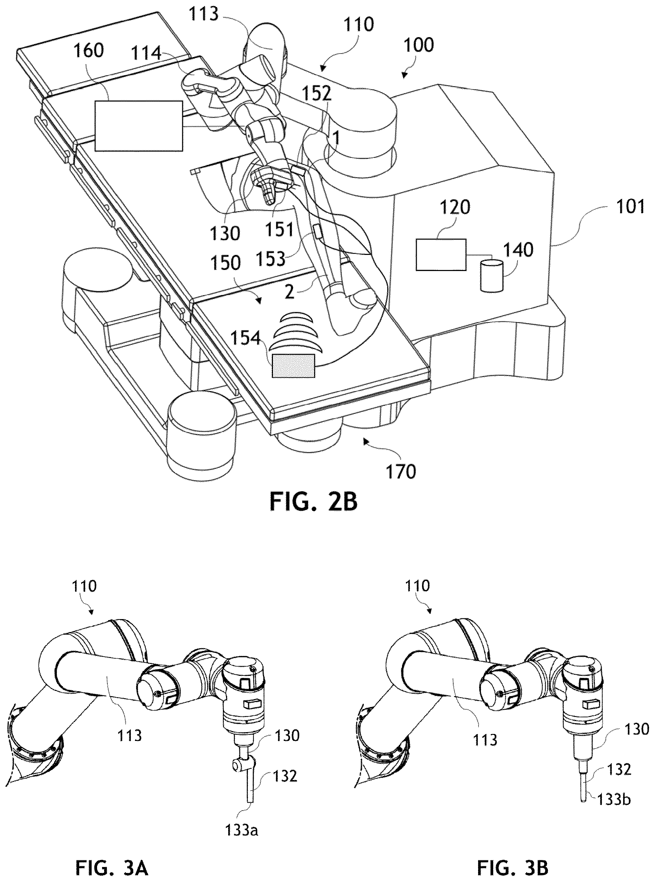

A surgical system 100 configured to perform the osteotomy and illustrated by several examples in figure 2a and 2b is described below.

The surgical system comprises a base from which extends a robotic arm 110. For instance, the base can be a wheeled cart 101 adapted to be displaced in the operating room.

The robotic arm extends between a first end connected to the base 101 and a second end which forms a flange of the robotic arm 110. The robotic arm 110 comprises several segments 113 connected to one another thanks to motorized joints 114. The robotic arm may comprise at least six motorized joints. The robotic arm could also comprise more than six motorized joints without departing from the scope of the disclosure.

One or more surgical tools may be interchangeably attached to the flange of the robotic arm to cut the bone according to a target axis or a target plane, the robotic arm constraining the tool in said target axis or target plane. In particular, at least one surgical tool may be configured to drill screw holes in the first bone. As shown in detail in figures 2a and 2b, the surgical tool 130 comprises a cutting tool 132.The same or another surgical tool 130 may be configured to perform a partial cut in the first bone to create the first and second bone portions. For instance, the cutting tool(s) 132 may be a saw (figure 3a), a drill (figure 3b), a burr, a laser, a water jet, a scalpel, focused ultrasounds, and/or a shaver.

By displacing the robotic arm, it is thus possible to drill a plurality of screw holes according to a planned sequence and/or to perform a plurality of partial cuts.

The surgical system comprises a control unit 120 configured to control the robotic arm. The control unit may, for instance, comprise one or more microprocessor, one or more random access memory (RAM) and/or one or more read-only memory (ROM), one or more calculators, one or more computers and/or one or more computer programs. The computer program(s) comprise code instructions to control the robotic arm. In addition, the control unit may include other devices and circuitry for performing the functions described herein such as, for example, a hard drive, input/output circuitry, and the like. The input/output circuitry can be adapted to treat digital and/or analog signals such as electromagnetic signals or video.

The surgical system can comprise at least one input device - not shown - and at least one processing unit adapted to receive inputs transmitted by the user through the input device and to compute instructions to be sent to one or several motorized joints of the robotic arm so as to activate and/or displace the surgical tool as commanded by the user, but with safety parameters - not detailed in this document - considered by said processing unit. For instance, the input device can be formed as a joystick or a handle mounted on the robotic arm, near the surgical tool. The processing unit may be integrated in the control unit or configured to communicate with the control unit.

The surgical system also comprises a tracking system 150. The tracking system is configured to determine, at a predetermined frequency, for example greater than 100 Hz, a relative pose of the robotic arm with respect to the first and the second bone, and to send corresponding localization data to the control unit. To that end, the tracking system comprises at least a first tracker attached to the robotic arm, a second and a third tracker respectively fixed to the first and second bones. The first tracker may be attached to the flange of the robotic arm. Alternatively, the first tracker may be arranged on any segment of the robotic arm. According to another alternative, the first tracker may be arranged on the base of the surgical system. A kinematic model of the robotic arm is stored in a memory of the control unit, and the control unit is thus able to determine the pose of the surgical tool relative to the first tracker based on said kinematic model.

The first and second trackers may be fixed respectively to the first and second bones by screwing or impacting.

The tracking system 150 may be an optical tracking system as for instance illustrated in figure 2a. The optical tracking system comprises at least a first tracker 151 rigidly fixed to the robotic arm 110, a second and third trackers 152, 153 rigidly attached respectively to the first and second bones 1, 2 and a camera system 154 configured to detect the current pose of the first, second and third trackers 151, 152, 153 and to determine, based on said current poses of the trackers 151, 152, 153, the relative pose of the robotic arm 110 with respect to the first and second bone 1, 2.

In another embodiment, the tracking system 150 may be an electromagnetic tracking system, as for instance illustrated in figure 2b.

The electromagnetic tracking system comprises at least a first, second and third trackers 151, 152, 153 configured to receive an electromagnetic field, and an emitting device 154 configured to emit the electromagnetic field. The tracking system is configured to determine, from the electromagnetic field received by the trackers 151, 152, 153, the relative pose of the robotic arm 110 with respect to the first and second bones 1, 2.

The surgical system also comprises a storage unit 140, the control unit being configured to communicate with the storage unit. This communication can be wired or wireless without departing from the scope of the disclosure. According to the illustrated embodiment, the control unit 120 and the storage unit 140 are embedded within the base 101 of the system as shown in figures 2a and 2b, but they could be positioned anywhere else within the system, or even be remote from said system. The storage unit is configured to store a 3D model of the first bone and the second bone.

The 3D model of the first bone is determined during a planning procedure, for example by the control unit, and represents a targeted deformation of the first bone to correct the misalignment of the limb. Therefore, the 3D model comprises a pose of the implant and of the plurality of screws to fix the implant. Several known methods can be used to carry out such planning and they are not developed in this document.

The control unit is further configured to compute a drilling axis for each screw and a cutting plane for each partial cut based on the 3D model of the first bone, for example during the planning procedure. Indeed, because the pose of the implant and the pose of the plurality of screws in the 3D model are already known, it is possible for example to compute the pose of each corresponding drilling axis. The control unit is also configured to determine a plurality of drilling axes and cutting planes when several screw holes and partial cuts are performed. The control unit is also configured to control the robotic arm to align the surgical tool with the predetermined cutting plane or the predetermined drilling axis based on the localization data received from the tracking system. By "aligning" is meant in the present text that an active portion of the surgical tool is within the cutting plane or the drilling axis. By "active portion" we here mean the portion of the surgical tool which is configured to cut or drill the region of interest. In the example illustrated in figure 3a wherein the surgical tool 130 is a saw, the active portion is formed by a blade 133a of the saw. In the example illustrated in figure 3b wherein the surgical tool 130 is a drill, the active portion is formed by the surface 133b of the drill bit. The control unit is configured to compute movement(s) and send instructions to control one or several motorized joints of the robotic arm to align the surgical tool with the predetermined cutting plane or the predetermined drilling axis based on the localization data. Several known methods can be used to carry out alignment and they are not developed in this document.

The alignment of the surgical tool can be done automatically by the robotic arm based on the planning and on localization data provided by the tracking system.

Alternatively, the alignment of the surgical tool can be commanded by the user via the input device, the control unit computing the instructions to the motorized joints based on the user's input, the planning and the localization data provided by the tracking system.

In order to allow the user of the system to operate the surgical tool safely, the control unit 120 may be further configured to allow an activation of the surgical tool 130 only when the surgical tool 130 is aligned with the predetermined cutting plane or the predetermined drilling axis.

The surgical tool can for instance be activated and/or guided by the user, through the input device. Alternatively, the control unit can be configured to activate the surgical tool to cut the bones automatically, based on the planning and on the determined poses of the concerned bones obtained thanks to the tracking system.

In order to drill a screw hole with a predetermined length, the control unit is also configured to compute a depth of the screw hole based on the 3D model, for example during the planning procedure. Indeed, because the plurality of screws in the 3D model are already known, it is possible to compute the pose of each corresponding depth. Therefore, the control unit is also configured to displace the surgical tool along the drilling axis until reaching the predetermined depth. Consequently, the surgical system does not drill further than a "virtual end limit" (i.e., the predetermined depth) which prevents overdrilling risks such as drilling soft tissues. Another advantage is that the user of the system can operate the surgical tool with higher precision by drilling until the proper depth in addition to the proper pose.

Each predetermined drilling axis, predetermined depth and predetermined cutting plane computed are also stored in the storage unit.

The surgical system may be configured to allow the surgeon to select easily and quickly a proper screw among a lot of screw references. To do that, the storage unit may also comprise a plurality of screw references, such as a screw database, each screw reference defining a type (e.g. mono-cortical or bi-cortical), a diameter and a length of a respective screw. The control unit is further configured to select, for the screw hole, a screw reference whose diameter is less than or equal to a diameter of the screw hole and whose length corresponds to the predetermined depth of the screw hole. It is therefore not necessary for the user to test, and thus waste, several screws in the screw hole until finding a proper screw reference.

Mono-cortical or bi-cortical screws may be used to fix the implant.

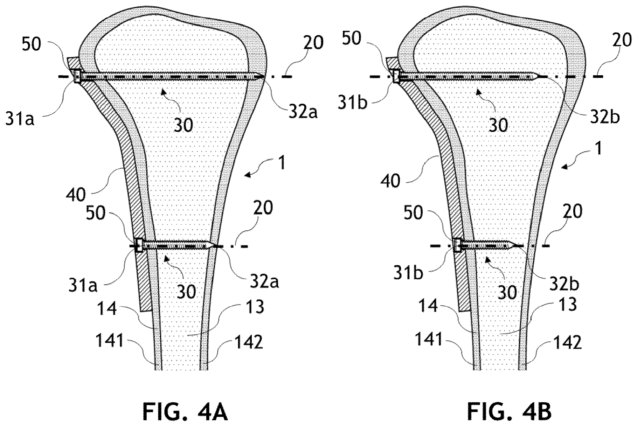

The first bone 1 comprises a cortical bone 14 and a cancellous bone 13 as illustrated in figures 4a and 4b. The cortical bone 14 is a hard part of the first bone 1 compared to the cancellous bone 13 and the cortical bone 14 surrounds the cancellous bone 13. The cancellous bone 13 is a spongy and porous part of the first bone 1 compared to the cortical bone 14. The control unit may be further configured to determine a bone volume comprising the cortical and the cancellous bone 13, 14 and a bone surface delimiting the bone volume. Therefore, the 3D model may further comprise the bone volume and the bone surface determined by the control unit.

The control unit may be further configured to determine a second bone surface delimiting a contact between the cortical bone 14 and the cancellous bone 13. Therefore, the 3D model may further comprise the second bone surface determined by the control unit.

In one embodiment, the control unit may determine the second bone surface by processing an image comprising the first and the second bones. The image may be a 2D image or a 3D image, for example obtained by X-rays or magnetic resonance imaging (MRI). Based on the 2D image or the 3D image, the control unit may also be configured to compute a first bone density and a second bone density corresponding respectively to the cortical bone and the cancellous bone in the 3D bone model. For example, shades of gray of the 2D image or the 3D image are processed by algorithms to delimit a plurality of areas according to different gray levels. Thus, the cortical bone appears in the 2D image or the 3D image brighter than the cancellous bone. Then, algorithms draw a line in the 3D bone model between these two different grey levels.

In another embodiment, the control unit may determine the second bone surface by a statistical method.

By "bi-cortical screw" we here mean a screw 50 engaged in two opposite regions forming the cortical bone 14 and in the cancellous bone (figure 4a). By "mono-cortical screw" we here mean a screw 50 engaged in one region forming the cortical bone 14 (i.e. the region closest to the implant) and in the cancellous bone 13 (figure 4b).

In the case of a bi-cortical screw, i.e., a screw engaged in two opposite regions 141, 142 forming the cortical bone 14, the control unit may be further configured to determine the predetermined depth as a distance along the predetermined drilling axis 20 from an entry point of the surgical tool on the bone surface to an end point located in the cortical bone 14 tissue opposite to the entry point with respect to the cancellous bone 13. Consequently, the surgical system 130 can perform the screw hole 30 delimited by the entry point 31a and the end point 32a without drilling through both sides of the first bone 1 as shown in figure 4a.

In the case of a mono-cortical screw, i.e. a screw engaged once in one region 141 forming the cortical bone 14 of the first bone 1, the control unit may also be configured to determine the predetermined depth as a distance along the predetermined drilling axis from the entry point of the surgical tool on the bone surface to an end point 32b located in the cancellous bone 13. Therefore, the surgical system 130 can perform properly the screw hole 30 delimited by the entry point 31b and the end point 32b as shown in figure 4b.

In addition to posing the surgical tool, the control unit may be further configured to determine a maximum and/or a minimum relative speed of the surgical tool with respect to the predetermined cutting plane or the predetermined drilling axis based on the localization data provided by the tracking system. Of course, a plurality of maximum and/or minimum relative speeds may be determined so as to adapt the relative speed of the surgical tool as required by the surgeon. For example, when the surgical tool is remote to the cutting plane or the drilling axis, the robotic arm needs to displace the surgical tool quickly. When the surgical tool is close to the cutting plane or the drilling axis, the robotic arm needs more accuracy and therefore needs to displace the surgical tool at a lower speed but also above a minimum speed to cut or drill accurately.

In one embodiment, the minimum relative speed is zero.

Therefore, the control unit may compute instructions to control the surgical tool based on the maximum and/or the minimum relative speed previously determined.

The control unit may also be configured to determine a maximum and/or a minimum rotation speed of the surgical tool based on the localization data provided by the tracking system. The rotation speed may be an oscillation of a saw or a rotation of a drill. The maximum and/or minimum rotation and relative speeds allow the user to drill and perform partial cuts without damage the first bone, for example by overheating bone tissues or by vibrations. Of course, a plurality of maximum and/or minimum rotation speeds may be determined so as to adapt the rotation speed of the surgical tool as required by the surgeon. Indeed, cancellous and cortical bones does not have a same hardness. In addition, cancellous bone does not overheat at a same temperature than the cortical bone. Therefore, determining different maximum and/or minimum rotation speeds allows to improve accuracy when drilling is performed.

In one embodiment, the minimum rotation speed is zero.

Therefore, the control unit may compute instructions to control the surgical tool based the maximum and/or the minimum rotation speed previously determined.

The control unit may be further configured to determine the relative speed and the rotation speed based on a size of the surgical tool, for example a length of the surgical tool, and a design of the surgical system, for example specifications of the motorized joints.

Finally, the surgical system advantageously comprises at least one user interface configured to display a representation computed by the control unit of the pose of the surgical tool with respect to each predetermined cutting plane and/or with respect to the predetermined drilling axis, a representation of the predetermined drilling axis, a representation of the predetermined depth, a representation of the predetermined cutting plane, and/or the 3D model. Thus, the user of the system can appreciate at any time the relative pose of the above objects with respect to one another. The user interface may be further configured to display, for each screw hole, a representation of the selected screw reference. Thus, the user can select a proper screw quickly. According to the illustrated embodiment in figures 2a and 2b, the user interface 160 is formed as a display attached to the robotic arm 110. Obviously, this is merely an example and the display could be positioned on another part of the surgical system, such as its base, within the scope of the disclosure. Alternatively, the display could also be attached to an operating table 170 where a patient lies in figure 2a and 2b. The display may integrate a touch-sensitive interface to receive inputs transmitted by the user. The display may be remoted controlled by the control unit. According to yet another alternative, the display device could be an augmented reality device configured to be positioned between the eyes of the user and the region of interest, such as augmented reality glasses, goggles, or panel.

## Claims

1. A surgical system (100) for osteotomy of a first bone (1) of a lower limb to correct a misalignment of the lower limb by moving a first bone portion (11) of the first bone and a second bone portion (12) of the first bone (1) away or toward each other about a bony hinge (13) to reach a targeted deformed configuration of the first bone (1) achieving a corrected alignment of the lower limb, the first bone portion (11) and the second bone portion (12) being created after cutting the first bone (1), the targeted deformed configuration of the first bone (1) being maintained by an implant (40) attached to the first bone portion (11) and the second bone portion (12) of the first bone (1) with a plurality of screws (50) in a plurality of respective screw holes, the lower limb comprising the first bone (1) and a second bone (2) articulated with the first bone (1),
the surgical system (100) comprising:
a storage unit (140);
a control unit (120) configured to communicate with the storage unit (140);
a robotic arm (110), the control unit (120) being configured to control the robotic arm (110);
a surgical tool (130) configured to be attached to the robotic arm (110) and to perform at least one of the following operations:
drilling at least one screw hole in the first bone (1) along a respective predetermined drilling axis (20) and until a respective predetermined depth,
performing a partial cut in the first bone (1) along at least one predetermined cutting plane to create the first bone portion (11) and the second bone portion (12),
the control unit (120) being configured to compute the respective drilling axis (20), the respective predetermined depth and the predetermined cutting plane based on a planned position of the implant (40) and a planned pose of the plurality of screws (50) on a 3D model, the 3D model representing the first bone (1) in the targeted deformed configuration,
the predetermined drilling axis, the predetermined depth and the predetermined cutting plane being stored in the storage unit (140),
a tracking system (150) comprising, at least, a first tracker (151) configured to be fixed to the robotic arm, a second tracker (152) and a third tracker (153) configured to be fixed respectively to the first bone (1) and the second bone (2), the tracking system (150) being configured to determine a relative pose of the robotic arm (110) with respect to the first and the second bone, and to send corresponding localization data to the control unit (120),
the control unit (120) being configured to control the robotic arm (110) to:
align the surgical tool (130) with the predetermined cutting plane or the predetermined drilling axis based on the localization data provided by the tracking system (150); and
displace the surgical tool (130) along the predetermined drilling axis (20) until reaching the predetermined depth based on the localization data.

2. The surgical system (100) of claim 1, wherein the 3D model further comprises a bone volume and a bone surface of the first bone (1), the bone volume comprising a cortical bone (14) and a cancellous bone (13) of the first bone (1), the bone surface delimiting the bone volume, and the control unit (120) is further configured to compute the predetermined depth as a distance along the predetermined drilling axis from an entry point (31a, 32a) of the surgical tool (130) on the bone surface to an end point (31b, 32b) located in the cortical bone (14) opposite to the entry point with respect to the cancellous bone (13).

3. The surgical system (100) according to one of the claims 1 to 2, wherein the storage unit (140) comprises a plurality of screw references, each screw reference defining a diameter and a length of a respective screw,
the control unit (120) being further configured to select, for each screw hole, a screw reference whose diameter is less than or equal to a diameter of the screw hole and whose length corresponds to the respective predetermined depth of the screw hole.

4. The surgical system (100) according to one of the claims 1 to 3, further comprising a user interface (160) coupled to the control unit (120),
the control unit (120) being further configured to compute a representation of a relative pose of the surgical tool with respect to the predetermined cutting plane or with respect to the predetermined drilling axis (20), a representation of the predetermined drilling axis (20), a representation of the predetermined depth and/or a representation of the predetermined cutting plane on the model of the first bone,
the user interface (160) being configured to display the representation.

5. The surgical system (100) according to claim 4 in combination with claim 3, wherein the user interface (160) is further configured to display, for each screw hole, a representation of the selected screw reference.

6. The surgical system (100) according to one of the claims 1 to 5, wherein the control unit (120) is further configured to allow an activation of the surgical tool (130) only when the surgical tool (120) is aligned with the predetermined cutting plane or the predetermined drilling axis (20).

7. The surgical system (100) according to one of the claims 1 to 6, wherein the control unit (120) is further configured to control the robotic arm (110) to displace and to align the surgical tool (130) at a relative speed with respect to the predetermined cutting plane or the predetermined drilling axis (20) between a predetermined minimum relative speed and a predetermined maximum relative speed.

8. The surgical system (100) according to one of the claims 1 to 7, wherein the control unit (120) is further configured to control the robotic arm (110) to displace and to align the surgical tool (130) at a rotation speed between a predetermined minimum rotation speed and a predetermined maximum rotation speed.

9. The surgical system (100) according to one of the claims 1 to 8, wherein the robotic arm (110) comprises at least six motorized joints (114), the control unit (120) being further configured to control a motorized joint (114) of the at least six motorized joints (114).

10. The surgical system according to one of claims 1 to 9, further comprising an input device configured to communicate with the control unit, the control unit (120) being configured to receive an input from a user through the input device and to take into account said input to control the robotic arm (110) and/or the surgical tool (130).
